(19) Europäisches Patentamt
European Patent Office
Office européen des brevets

(11) **EP 2 335 669 A2**

(12) **EUROPEAN PATENT APPLICATION**
published in accordance with Art. 153(4) EPC

(43) Date of publication:
**22.06.2011 Bulletin 2011/25**

(51) Int Cl.:
*A61K 6/08* (2006.01)

(21) Application number: **09819391.5**

(22) Date of filing: **08.10.2009**

(86) International application number:
**PCT/KR2009/005760**

(87) International publication number:
**WO 2010/041884 (15.04.2010 Gazette 2010/15)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **08.10.2008 KR 20080098832**

(71) Applicant: **Seoul National University R&DB Foundation**
**Seoul 151-050 (KR)**

(72) Inventors:
• **PARK, Yoon-Jeong**
**Seoul 152-770 (KR)**

• **CHUNG, Chong-Pyoung**
**Seoul 138-170 (KR)**
• **LEE, Jue-Yeon**
**Gwacheon-si**
**Gyeonggi-do 427-736 (KR)**
• **PARK, Hyun Jung**
**Seoul 132-773 (KR)**

(74) Representative: **Albrecht, Thomas**
**Kraus & Weisert**
**Patent- und Rechtsanwälte**
**Thomas-Wimmer-Ring 15**
**80539 München (DE)**

(54) **COMPOSITION FOR TOOTH DESENSITIZER**

(57) The present invention relates to a composition for prevention of tooth hypersensitivity, and more particularly to a composition for prevention of tooth hypersensitivity, which comprises an adhesive polymer for coating teeth, an active ingredient for prevention of hypersensitivity, a substance for promoting the penetration of the active ingredient for prevention of hypersensitivity, a desensitizing active ingredient, and a pH-adjusting agent. The composition for prevention of tooth hypersensitivity according to the invention can rapidly show the effect of preventing tooth hypersensitivity while maintaining the sensory feel of conventional tooth desensitizers, and it can maintain the tooth hypersensitivity-preventing effect for a long time.

FIG. 2

**Description**

**TECHNICAL FIELD**

The present invention relates to a composition for prevention of tooth hypersensitivity, and more particularly to a composition for prevention of tooth hypersensitivity, which comprises an adhesive polymer for coating teeth, an active ingredient for prevention of hypersensitivity, a substance for promoting the penetration of the active ingredient for prevention of hypersensitivity, a desensitizing active ingredient, and a pH-adjusting agent.

**BACKGROUND ART**

The hard tissues of teeth include enamel, cement and dentine. Enamel covers the outside of the tooth which is exposed to the oral cavity, cement covers the outside of the tooth root located in the alveolar bone, and dentine is present inside the enamel and the cement.

Cement is weaker than enamel so that it is easily worn by excessive tooth brushing. Dentine contains dentinal tubules distributed throughout it. In the dentinal tubules, processes of dental pulp cells run from the dental pulp to enamel, and the space between the dentinal tubules and the cell processes is filled with dentinal fluid.

Tooth hypersensitivity indicates that, when eating sour fruits or drinking cold drinks, the teeth feel sensitive, or this condition is long-lasting or, in severe cases, is developed into a pain. In most cases, the causes of hypersensitivity are dentinal tubules and their contents, and the symptoms of hypersensitivity appear when the entrance of dentinal tubules is exposed. The most frequent case, the entrance of dentinal tubules is exposed because enamel and cement are destroyed due to incorrect tooth brushing. In some cases, the entrance of dentinal tubules is exposed by gingival recession due to periodontitis.

If enamel or cement is destroyed by any cause, the entrance of dentinal tubules will exposed to the outside, and irritation will be transferred to the dental pulp by the cell processes of dentinal tubules, nerves and dentinal fluid. If the irritation caused by cold drinks or sour fruits is transferred to the dental pulp through dentinal tubules, the dental pulp will feel sour and feel pain in severe cases.

Accordingly, the essential point of the treatment of tooth hypersensitivity is to occlude the entrance of exposed dentinal tubules to prevent irritation from being transferred to the dental pulp. There are various methods for treating tooth hypersensitivity, and a suitable treatment method can be selected depending on the degree of destruction of enamel and cement and the degree of symptoms.

In the case where the destruction of enamel and cement is severe, if the entrance of exposed dentinal tubules is occluded, followed by plaque control, the symptoms thereof will disappear or be relieved in most cases. Where the destruction of enamel or cement is insignificant, the relationship between the gum and the teeth has been deteriorated due to the destruction of the form of the tooth, so that periodontal disease has occurred or can occur in future. Accordingly, in this case, it is preferable to use a method of reinstate the original form of the tooth while occluding the entrance of exposed dentinal tubules.

At present, the best method is to reinstate the original form of the tooth while occluding the entrance of exposed dentinal tubules. In this case, it is preferable to use a composite resin, which is anesthetically natural, has strong adhesive strength and can minimize the additional deletion of teeth. The use of amalgam in place of the composite resin cannot be considered as a good method, because it shows poor aesthetic properties and the additional deletion of teeth is required so that the original form of teeth can be reinstated.

The above-described treatment method which is currently being used is expensive and inconvenient for a patient to visit a hospital. For this reason, there is an increasing demand for an easy-to-use and cost-effective means for treatment of tooth hypersensitivity which can be used by patients even at home.

Meanwhile, it has been reported through patent documents that hydroxyapatite (German Patent No. 2,134,862), strontium chloride (US Patent No. 3,122,483), potassium nitrate (US Patent No. 4,357,318) can be used as tooth desensitizing agents for treatment of hypersensitive teeth.

In addition, salts, including potassium hydroxide, magnesium hydroxide, sodium chloride, calcium phosphate, silver nitrate and sodium citrate, have been used as tooth desensitizing agents. It is known that toothpaste compositions containing said salts as tooth desensitizing agents act to desensitize hypersensitive teeth, because said salts are absorbed into exposed dentine so that they surround nerve fiber tissue while acting as a buffer.

Particularly, according to the reports of John W. Federn (Journal of Periodontology, American East Area, Vol. 25, No. 2 (Summer, 1997)), Bareire Green et al. (American Journal of Periodontology, Vol. 48, No. 10 (Oct., 1977)), Willard J. Tablet et al. (American Journal of Periodontology, Vol. 51, No. 9, (Sep., 1980) and Milton Hodosh (Journal of the American Dental Association, Vol. 88 (Apr. 1974)), it can be seen that potassium nitrate has the most excellent effect among tooth desensitizing agents which are used in toothpastes.

In recent years, as patients with hypersensitive teeth increased, it is urgent to develop a material which has an excellent effect of preventing tooth hypersensitivity compared to existing tooth hypersensitivity-treating agents and can also exhibit an effect of preventing tooth decay.

Accordingly, the present inventors have made many efforts to provide an improvement over the conventional agents which have been used for treatment of tooth hypersensitivity in the prior art. As a result, the present inventors have found that a composition for preventing tooth hypersensitivity, which comprises an adhesive polymer for dental use, an active ingredient for prevention of tooth hypersensitivity, a substance for promoting the penetration of the active ingredient for prevention of tooth hypersensitivity, a desensitizing active ingredient and a pH-adjusting agent, can rapidly show the effect of preventing tooth hypersensitivity while maintaining the sensory feel of the conventional tooth hypersensitivity-treating agents and can maintain the tooth hypersensitivity-preventing effect for a long time, thereby completing the present invention.

**DISCLOSURE OF INVENTION**

It is an object of the present invention to provide a composition for prevention of tooth hypersensitivity, which rapidly shows the effect of preventing tooth hypersensitivity and can maintain the tooth hypersensitivity-preventing effect for a long time.

To achieve the above object, the present invention provides a composition for prevention of tooth hypersensitivity, which comprises an adhesive polymer for coating teeth, an active ingredient for prevention of hypersensitivity, a substance for promoting the penetration of the active ingredient for prevention of hypersensitivity, a desensitizing active ingredient and a pH-adjusting agent.

The present invention also provides a method for preparing a composition for prevention of tooth hypersensitivity, the method comprising the steps of: (a) dissolving in a volatile solvent an adhesive polymer for coating teeth to form an adhesive polymer solution; (b) mixing the adhesive polymer solution of step (a) with an active ingredient for prevention of hypersensitivity, a substance for promoting the penetration of the active ingredient for prevention of hypersensitivity and a desensitizing active ingredient to form a mixture; (c) adding a pH-adjusting agent to the mixture of step (b) to adjust the pH of the mixture; and (d) filtering the pH-adjusted mixture of step (c) to remove impurities.

**BRIEF DESCRIPTION OF THE DRAWINGS**

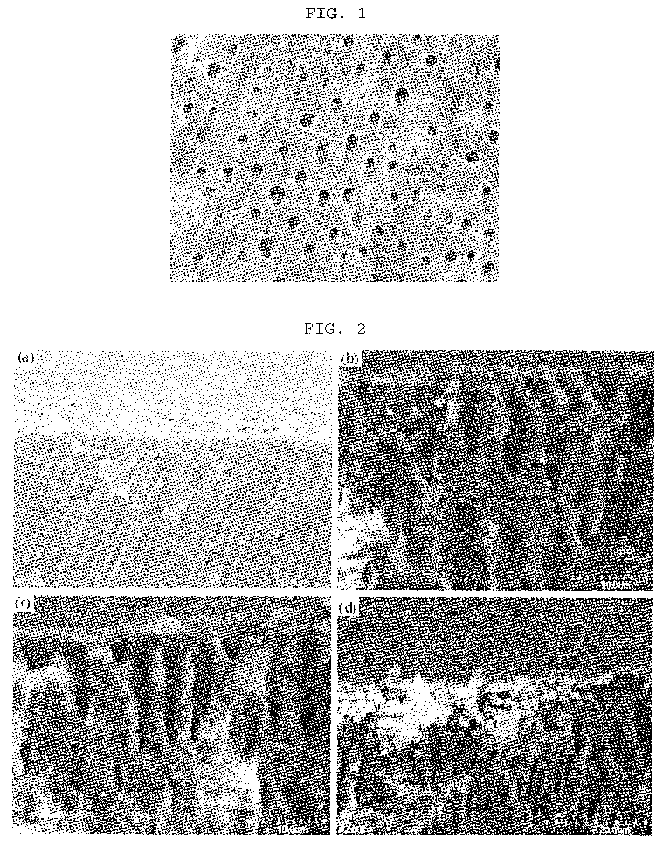

FIG. 1 is a photograph showing the result of observing a tooth surface with a scanning electron microscope after etching the tooth surface with 6% citric acid.

FIG. 2 is a set of scanning electron micrographs showing the results obtained by sectioning a tooth vertically, etching the tooth surface with 6% citric acid, applying the inventive composition for prevention of tooth hypersensitivity to the tooth and observing the tooth with a scanning electron microscope ((a): 1000x magnification, (b) and (c): 3000x magnification, and (d): 2000x magnification).

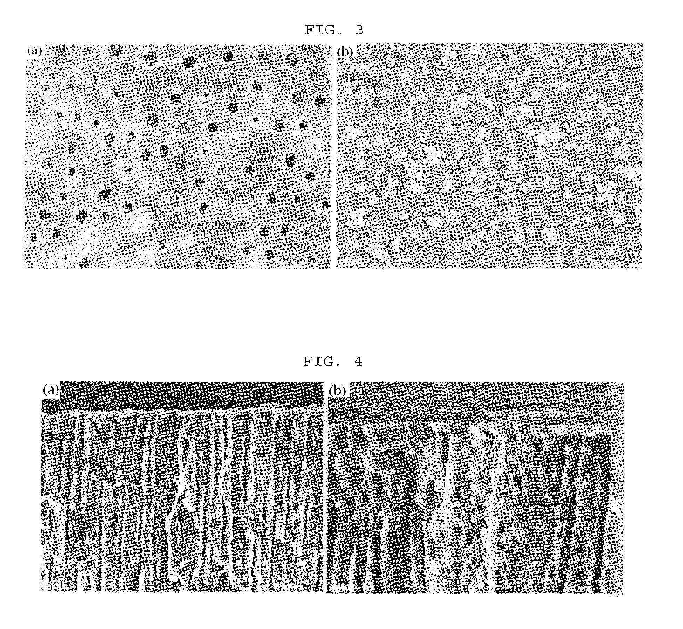

FIG. 3(a) is a scanning electron micrograph showing the result obtained by etching a tooth surface with 6% citric acid and observing the etched tooth surface with a scanning electron microscope, and FIG. 3(b) is a scanning electron micrograph showing the result obtained by applying the inventive composition for prevention of tooth hypersensitivity to the etched tooth surface of FIG. 3(a), and then observing the tooth surface with a scanning electron microscope.

FIG. 4(a) is a scanning electron micrograph showing the result obtained by sectioning a tooth vertically, etching the tooth surface with 6% citric acid and observing the etched tooth surface with a scanning electron microscope, and FIG. 4(b) is a scanning electron micrograph showing the result obtained by applying the inventive composition for prevention of tooth hypersensitivity to the etched tooth surface of FIG. 4(a), and then observing the tooth surface with a scanning electron microscope.

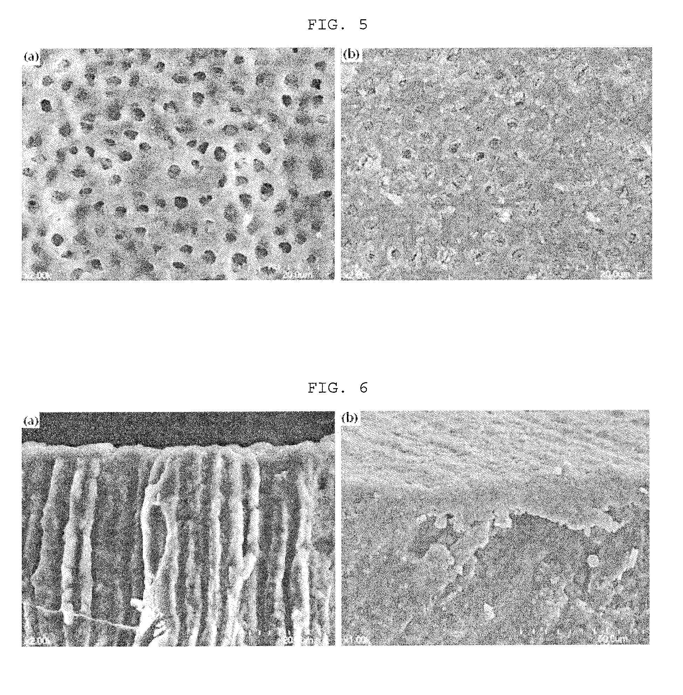

FIG. 5(a) is a scanning electron micrograph showing the result obtained by etching a tooth surface with 6% citric acid and observing the etched tooth surface with a scanning electron microscope, and FIG. 5(b) is a scanning electron micrograph showing the result obtained by applying SuperSeal (Phoenix Dental, USA) to the etched tooth surface of FIG. 5(a) and then observing the tooth surface with a scanning electron microscope.

FIG. 6 (a) is a scanning electron micrograph showing the result obtained by sectioning a tooth vertically, etching the tooth surface with 6% citric acid and then observing the etched tooth surface with a scanning electron microscope, and FIG. 6(b) is a scanning electron micrograph showing the result obtained by applying SuperSeal (Phoenix Dental, USA) to the etched tooth surface of FIG. 6(a) and then observing the tooth with a scanning electron microscope.

## DETAILED DESCRIPTION OF THE INVENTION AND

## BEST MODE FOR CARRYING OUT THE INVENTION

In one aspect, the present invention is directed to a composition for prevention of tooth hypersensitivity, which comprises an adhesive polymer for coating teeth, an active ingredient for prevention of hypersensitivity, a substance for promoting the penetration of the active ingredient for prevention of hypersensitivity, a desensitizing active ingredient, and a pH-adjusting agent.

In the present invention, the composition for prevention of tooth hypersensitivity preferably comprises, based on 100 parts by weight of the adhesive polymer for coating teeth, 2-3 parts by weight of the active ingredient for prevention of hypersensitivity, 2-10 parts by weight of the substance for promoting the penetration of the active ingredient for prevention of hypersensitivity, 4-10 parts by weight of the desensitizing ingredient and 2-3 parts by weight of the pH-adjusting agent.

As used herein, the term "adhesive polymer for coating teeth" refers to a polymer which has adhesion to teeth when being hydrated and is coated on the tooth surface to form a film.

In the present invention, the adhesive polymer for coating teeth is preferably one or more selected from the group consisting of hydroxypropyl methyl cellulose, hydroxyethyl cellulose, polyvinyl alcohol, polyvinyl pyrrolidone, hydroxypropyl cellulose, polyacrylate derivative carbomer, a copolymer of methyl vinyl ether and maleic anhydride, and polyethylene oxide.

In the present invention, the adhesive polymer for coating teeth preferably has a weight-average molecular weight of 13,000-4,000,000, and more preferably 25,000-2,000,000. If the weight-average molecular weight of the adhesive polymer for coating teeth is less than 13,000, the polymer will hardly reach a viscosity suitable for application when it is used in a conventional amount. If the weight-average molecular weight is more than 4,000,000, the polymer will have a high viscosity even at low concentration, which makes it difficult to apply the polymer.

In the present invention, the polyvinyl pyrrolidone is preferably one or more selected from the group consisting of polyvinyl pyrrolidone K-12, polyvinyl pyrrolidone K-15, polyvinyl pyrrolidone K-17, polyvinyl pyrrolidone K-25, polyvinyl pyrrolidone K-30, polyvinyl pyrrolidone K-60, polyvinyl pyrrolidone K-90 and polyvinyl pyrrolidone K-120. Herein, "K" described above with respect to the polyvinyl pyrrolidine means a numerical value that generally appears in proportion to the molecular weight (chain length, polymerization degree) of the polyvinyl pyrrolidone. As the molecular weight increases, the viscosity increases and the K value also increases. The K values are described in the United States Pharmacopeia, and the physical and chemical properties of the polyvinyl pyrrolidones are described in the United States Pharmacopeia and the Handbook of Pharmaceutical Excipients.

The active ingredient for prevention of hypersensitivity which is used in the present invention is a substance that prevents tooth hypersensitivity by forming crystals at the entrance of dentinal tubules of teeth to occlude the fine pores of the tooth surface. The content of the active ingredient for prevention of hypersensitivity is preferably 2-10 parts by weight, and more preferably 2-3 parts by weight, based on 100 parts by weight of the adhesive polymer for coating teeth. If the content of the active ingredient for prevention of hypersensitivity is less than 2 parts by weight, it cannot achieve the effect of preventing hypersensitivity, and if it is more than 10 parts by weight, it can cause various problems, including tooth surface erosion and oral mucosal damage.

In the present invention, the active substance for prevention of hypersensitivity is preferably one or more selected from the group consisting of potassium nitrate, stannous fluoride ($SnF_2$), sodium monofluoride phosphate (MFP), sodium fluoride (NaF), Sodium silicofluoride ($Na_2SiF_6$), calcium hydroxide ($Ca(OFi)_2$), potassium oxalate ($K_2C_2O_4$) and strontium chloride ($SrCl_2$).

The substance for promoting the penetration of the active ingredient for prevention of hypersensitivity, which is used in the present invention, serves to allow the active ingredient for prevention of hypersensitivity to rapidly penetrate into the tooth surface, thereby occluding the dentinal tubules. The content of the substance for promoting the penetration of the active ingredient for prevention of hypersensitivity is preferably 2-5 parts by weight based on 100 parts by weight of the adhesive polymer for coating teeth. If the content of the substance for promoting the penetration of the active ingredient for prevention of hypersensitivity is less than 2 parts by weight, it cannot achieve a desired penetration effect, and if the content is more than 5 parts by weight, it can interfere with the penetration of the active ingredient, rather than promoting the penetration, thereby irritating the mucosa.

In the present invention, the substance for promoting the penetration of the active ingredient for prevention of hypersensitivity is preferably a peptide having an arginine of 80-90%.

In the present invention, the peptide having an arginine content of 80-90% is preferably one or more selected from the group consisting of TAT (trans-activator of transcription), penetratine, polylysine, polyarginine and protamine fragments.

The desensitizing active ingredient that is used in the present invention is a substance that desensitizes nerves to prevent tooth hypersensitivity. The content of the desensitizing active ingredient is preferably 4-10 parts by weight based on 100 parts by weight of the adhesive polymer for coating teeth. If the content of the desensitizing active ingredient is less than 4 parts by weight, it will show a weak ability to desensitize nerves, so that the effect of preventing tooth hypersensitivity will be insufficient. If the content is more than 10 parts by weight, an increase in the content will not lead to any further increase in the desensitizing effect.

In the present invention, the desensitizing active ingredient is preferably one or more selected from the group consisting of salts, potassium nitrate and strontium chloride, wherein the salts are preferably selected from the group consisting of potassium hydroxide, magnesium hydroxide, sodium chloride, calcium phosphate, silver nitrate and sodium citrate.

The pH-adjusting agent that is used in the present invention serves to adjust the pH of the composition for prevention of tooth hypersensitivity so as to improve the long-term stability of the composition. The content of the pH adjusting agent is preferably 2-3 parts by weight based on 100 parts by weight of the adhesive polymer for coating teeth. If the content of the pH-adjusting agent is less than 2 parts by weight, it cannot adjust the pH of the composition, and if it is more than 3 parts by weight, the increase in the content will not lead to any further increase in the effect and will increase the pH of the composition to an excessively alkaline pH value which is unfavorable for teeth or the gum.

In the present invention, the pH-adjusting agent is one or more selected from the group consisting of disodium phosphate, trisodium phosphate, sodium pyrophosphate, potassium pyrophosphate, sodium hydrogen carbonate, potassium hydroxide, ammonium hydroxide, triethanolamine, citric acid, sodium citrate, and sodium hydroxide.

In the present invention, the composition for prevention of tooth hypersensitivity preferably has a pH of 4.0-11.0. If the composition for prevention of tooth hypersensitivity has an acidic pH of less than 4.0, it can cause gum necrosis or tooth erosion, and if the composition has an alkaline pH of more than 11.0, it will be unfavorable for the gum or teeth.

In the present invention, the composition for prevention of tooth hypersensitivity may further comprise a sweetening agent and/or a flavoring agent.

The sweetening agent that is used in the present invention serves to improve the sensory feel of the composition and to eliminate a bitter taste which can be caused by other compositions. The content of the sweetening agent is preferably 2-10 parts by weight based on 100 parts by weight of the total weight of the composition. If the content of the sweetening agent is less than 2 parts by weight, it cannot give a sweet taste to the composition, and if the content is more than 10 parts by weight, it will give an excessively sweet taste which can be unpleasant.

In the present invention, the sweetening agent is preferably one or more selected from the group consisting of sorbitol, mannitol, xylitol, stevioside and saccharine.

The flavoring agent that is used in the present invention serves to improve the sensory feel of the composition for prevention of tooth hypersensitivity and to eliminate odors that can occur due to other compositions. The content of the flavoring agent is preferably 2-10 parts by weight based on 100 parts by weight of the adhesive polymer for coating teeth. If the content of the flavoring agent is less than 2 parts by weight, it cannot flavor to the composition, and if the content is more than 10 parts by weight, it will give an excessively strong flavor to the composition.

In the present invention, the flavoring agent is preferably one or more selected from the group consisting of menthol, peppermint oil, L-menthol and cinnamic acid.

In the present invention, the composition for prevention of tooth hypersensitivity is preferably in the form of gel or liquid, which can easily penetrate into the cracks of the tooth surface and can more efficiently adhere to the tooth.

In the present invention, the composition for prevention of tooth hypersensitivity is preferably prepared according to a method comprising the steps of: (a) dissolving in a volatile solvent an adhesive polymer for coating teeth to form an adhesive polymer solution; (b) mixing the adhesive polymer solution of step (a) with an active ingredient for prevention of hypersensitivity, a substance for promoting the penetration of the active ingredient for prevention of hypersensitivity and a desensitizing active ingredient to form a mixture; (c) adding a pH-adjusting agent to the mixture of step (b) to adjust the pH of the mixture; and (d) filtering the pH-adjusted mixture of step (c) to remove impurities.

The volatile solvent that is used in the present invention serves to dissolve the adhesive polymer for coating teeth and is preferably used in an amount of 200-240 parts by weight based on 100 parts by weight of the adhesive polymer for coating teeth. If the volatile solvent is used in an amount of less than 200 parts by weight, it cannot completely dissolve the adhesive polymer for coating teeth and will not rapidly volatilize when the composition is applied to teeth, thus making the use of the composition inconvenient. If the content of the volatile solvent is more than 240 parts by weight, the increase in the content will not lead to any further increase in the effect.

In the present invention, the volatile solvent that is used in step (a) of the inventive method is preferably one or more selected from the group consisting of ethanol, methanol and butanol.

The composition for prevention of tooth hypersensitivity according to the present invention has an improved effect of preventing tooth hypersensitivity by occluding dentinal tubules of teeth while desensitizing nerves. When the composition is applied to teeth, it will rapidly adhere to the teeth due to the substance for promoting the penetration of the active ingredient for prevention of hypersensitivity, so that the effect thereof will appear rapidly. In addition, the effect thereof can be lasting for a long time due to the action of the pH-adjusting agent.

**EXAMPLES**

Hereinafter, the present invention will be described in further detail with reference to examples.

It will be obvious to a person having ordinary skill in the art that these examples are illustrative purposes only and are not to be construed to limit the scope of the present invention.

**Example 1: Preparation of liquid composition for prevention of tooth hypersensitivity**

A liquid composition for prevention of tooth hypersensitivity was prepared by mixing the following components: polyvinyl alcohol and polyvinyl pyrrolidone as adhesive polymers for coating teeth; potassium oxalate as an active ingredient for prevention of hypersensitivity; a peptide having an arginine content of 80-90% as a substance for promoting the penetration of the active ingredient for prevention of hypersensitivity; potassium nitrate and strontium chloride as desensitizing active ingredients; KOH as a pH-adjusting agent; and 60% ethanol as a volatile solvent.

1-1. Step of dissolving in a volatile solvent an adhesive polymer for coating teeth

40 ml of distilled water and 0.5 g of 0.5% polyvinyl alcohol were mixed with each other, heated at 85~90 °C for 30 minutes and cooled. Then, 60 ml of 99.9% anhydrous ethanol was added thereto, thus preparing 100 ml of 60% ethanol as a volatile solvent.

Then, 2 g of polyvinyl pyrrolidone was dissolved in 100 ml of the 60% ethanol.

1-2. Step of mixing an active ingredient for prevention of hypersensitivity, a substance for promoting the penetration of the active ingredient for prevention of hypersensitivity and a desensitizing active ingredient

To 100 ml of the 60% ethanol containing 2 g of polypyrrolidone, prepared in Example 1-1, 0.05 g of potassium oxalate, 0.05 g of a peptide having an arginine content of 80-90%, 0.05 g of potassium nitrate and 0.05 g of strontium chloride were added, thus preparing a mixture.

1-3. Step of adjusting pH

The mixture prepared in Example 1-2 was adjusted to pH of 7.0 by adding KOH thereto.

1-4. Step of removing impurities

The pH-adjusted mixture of Example 1-3 was filtered through a 75-mm-diameter membrane filter (45 mm neck; NALGENE, USA), thereby preparing a composition for prevention of tooth hypersensitivity.

**Test Example 1: Test for non-vital tooth using liquid composition for prevention of tooth hypersensitivity prepared in Example 1.**

In order to evaluate whether the liquid composition for prevention of tooth hypersensitivity prepared in Example 1 functions as an agent for preventing or treating tooth hypersensitivity by occluding the fine pores of the tooth surface, the effect of the composition on the sealing of dentinal tubules of non-vital tooth was observed with a scanning electron microscope. Herein, the term "non-vital tooth" means a tooth whose dental pulp was dead by an external impact or tooth decay and penetrated into the fine pores of the tooth to discoloration so that the tooth was turned dark gray or black in severe cases because of the hemoglobin component of the dental pulp.

A non-vital back tooth which has not elapsed one after tooth extraction was immersed in distilled water of 4 °C containing 100 ml of 0.1 M cacodylate buffer and 3 ml of glutaraldehyde. The non-vital tooth was obtained from the Seoul National University Dental Hospital.

After 1 week, the non-vital tooth was taken out of the distilled water, and the enamel layer was removed. Then, the tooth was sectioned to a thickness of 1 mm, thus obtaining a dentinal disk. Then, the surface of the dentinal disk was etched with 6% citric acid for 2 minutes (FIGS. 1, 3(a) and 4(a)), after the disk was washed sufficiently with purified water and dried. To the surface of the dried disk, the liquid composition for prevention of tooth hypersensitivity prepared in Example was applied, after which the surface of the disk was observed with a scanning electron microscope (Field Emission Electron Microscope, S-4700, Hitachi, Japan) (FIGS. 2, 3(b) and 4(b)).

**Comparative Example 1: Test for non-vital tooth using SuperSeal (Phoenix Dental, USA)**

In order to evaluate whether SuperSeal (Phoenix Dental, USA) functions as an agent for preventing or treating tooth hypersensitivity by occluding the fine pores of the tooth surface, the effect of SuperSeal on the sealing of dentinal tubules of non-vital teeth was observed with a scanning electron microscope.

A non-decayed, non-vital back tooth which has not elapsed one month after tooth extraction was immersed in distilled water of 4 ˚C containing 100 ml of 0.1 M cacodylate buffer and 3 ml of 3% glutaraldehyde. The non-vital tooth was obtained from the Seoul National University Dental Hospital.

After 1 week, the non-vital tooth was taken out of the distilled water, and the enamel layer was removed. Then, the tooth was sectioned to a thickness of 1 mm, thus obtaining a dentinal disk. Then, the surface of the dentinal disk was etched with 6% citric acid for 2 minutes, after the disk was washed sufficiently with purified water and dried (FIGS. 5(a) and 6(a)). To the surface of the dried disk, SuperSeal (Phoenix Dental, USA) was applied, after which the surface of the disk was observed with a scanning electron microscope (Field Emission Electron Microscope, S-4700, Hitachi, Japan) (FIGS. 5(b) and 6(b)).

**Test Example 2: Comparison of ratio of occluded dentinal tubules between tooth hypersensitivity-preventing composition of Example 1 and SuperSeal (Phoenix Dental, USA)**

Photographs of FIGS. 3 and 5 were subjected to image analysis, after which the ratio of dentinal tubules occluded by each of the tooth hypersensitivity-preventing composition of Example and SuperSeal (Phoenix Dental, USA), a currently commercially available desensitizing agent, was calculated according to the following equation (I) using Tomoro Scope Eye (Tomoro, Korea) program:

```
    Ratio of occluded dentinal tubules = (sum of areas of
  occluded dentinal tubules / sum of areas of total dentinal
  tubules) x 100        ----------------------------------(I)
```

As a result, the ratio of dentinal tubules occluded by the tooth hypersensitivity-preventing liquid composition of Example 1 and the ratio of dentinal tubules occluded by SuperSeal (Phoenix Dental, USA) were 97.11% and 42.82%, respectively, indicating that the ratio of dentinal tubules occluded by the tooth hypersensitivity-preventing liquid composition of Example 1 was at least two times higher than that of SuperSeal (Phoenix Dental, USA).

This suggests that the composition for prevention of tooth hypersensitivity according to the present invention exhibits an excellent effect of preventing tooth hypersensitivity.

[Table 1]

| Results of image analysis of FIG. 3 | | |
|---|---|---|
| Measurement Table | | |
| Name | Length | Area |
| Polygon-210 | 410.83 | 8244.37 |
| Polygon-211 | 260.93 | 4425.31 |
| Polygon-212 | 319.66 | 7079.57 |
| Polygon-213 | 295.51 | 5705.51 |
| Polygon-214 | 422.63 | 11035.58 |
| Polygon-215 | 280.76 | 5677.81 |
| Polygon-216 | 356.96 | 9036.80 |
| Polygon-217 | 385.27 | 10015.42 |
| Polygon-218 | 348.66 | 7941.25 |

(continued)

| Results of image analysis of FIG. 3 | | |
| --- | --- | --- |
| Measurement Table | | |
| Name | Length | Area |
| Polygon-219 | 315.11 | 6737.98 |
| Polygon-220 | 341.27 | 7967.40 |
| Polygon-221 | 265.44 | 4634.57 |
| Polygon-222 | 339.92 | 8232.06 |
| Polygon-223 | 309.85 | 5613.19 |
| Polygon-224 | 260.57 | 4760.75 |
| Polygon-225 | 254.53 | 4266.82 |
| Polygon-226 | 226.19 | 3349.76 |
| Polygon-227 | 248.36 | 4336.07 |
| Polygon-228 | 157.65 | 1558.71 |
| Polygon-229 | 261.16 | 4102.18 |
| Polygon-230 | 253.87 | 3375.91 |
| Polygon-231 | 349.53 | 8092.04 |
| Polygon-232 | 297.48 | 6056.33 |
| Polygon-233 | 280.60 | 5607.03 |
| Polygon-234 | 265.66 | 4394.54 |
| polygon-235 | 318.92 | 7158.05 |
| Polygon-236 | 335.85 | 7550.42 |
| Polygon-237 | 212.69 | 2925.07 |
| polygon-238 | 296.97 | 6208.67 |
| Polygon-239 | 329.06 | 7479.64 |
| Polygon-240 | 300.31 | 6119.42 |
| Polygon-241 | 300.31 | 5747.06 |
| Polygon-242 | 304.77 | 6056.33 |
| Polygon-243 | 333.55 | 7398.08 |
| Polygon-244 | 323.69 | 7333.46 |
| Polygon-245 | 312.15 | 6173.28 |
| Polygon-246 | 358.86 | 9050.65 |
| Polygon-247 | 268.73 | 3811.37 |
| Polygon-248 | 306.07 | 6524.10 |
| Polygon-249 | 268.67 | 4491.47 |
| Polygon-250 | 211.81 | 2485.01 |
| Polygon-251 | 223.70 | 3402.07 |
| Polygon-252 | 248.69 | 3594.41 |
| Polygon-253 | 276.72 | 5103.88 |
| Polygon-254 | 241.27 | 3783.67 |

(continued)

| Results of image analysis of FIG. 3 | | |
|---|---|---|
| Measurement Table | | |
| Name | Length | Area |
| Polygon-255 | 224.55 | 3455.93 |
| Polygon-256 | 309.97 | 6739.52 |
| Polygon-257 | 304.92 | 6296.37 |
| Polygon-258 | 289.91 | 5530.10 |
| Polygon-259 | 200.05 | 2749.66 |
| Polygon-260 | 420.07 | 12488.11 |
| Polygon-261 | 303.67 | 6116.34 |
| Polygon-262 | 201.90 | 2605.02 |
| Polygon-263 | 358.61 | 8333.62 |
| Polygon-264 | 338.81 | 8110.50 |
| Polygon-265 | 429.43 | 11667.98 |
| Polygon-266 | 317.02 | 6814.92 |
| Polygon-267 | 249.63 | 4372.99 |
| Polygon-268 | 266.31 | 4988.48 |
| Polygon-269 | 323.29 | 7444.25 |
| Polygon-270 | 294.21 | 5813.22 |
| Polygon-271 | 322.43 | 6717.98 |
| Polygon-272 | 218.46 | 2915.84 |
| Polygon-273 | 314.90 | 6733.36 |
| Polygon-274 | 366.13 | 9544.57 |
| Polygon-275 | 200.01 | 2703.50 |
| Polygon-276 | 173.82 | 1820.29 |
| Polygon-277 | 421.97 | 11704.91 |
| Polygon-278 | 441.70 | 12543.51 |
| Polygon-279 | 266.34 | 4933.08 |
| Polygon-280 | 351.59 | 8942.94 |
| Polygon-281 | 285.32 | 5125.42 |
| Polygon-282 | 269.22 | 4960.78 |
| Polygon-283 | 379.34 | 9159.90 |
| Polygon-284 | 221.99 | 3380.53 |
| Polygon-285 | 234.62 | 3388.22 |
| Polygon-286 | 190.04 | 2223.43 |
| Polygon-287 | 231.10 | 3323.60 |
| Polygon-288 | 242.64 | 3288.21 |
| Polygon-289 | 177.94 | 1626.41 |
| Polygon-290 | 192.72 | 1869.52 |

(continued)

| Results of image analysis of FIG. 3 | | |
|---|---|---|
| Measurement Table | | |
| Name | Length | Area |
| Polygon-291 | 207.87 | 2760.43 |
| Polygon-292 | 249.66 | 3749.82 |
| Polygon-293 | 289.94 | 5651.66 |
| Polygon-294 | 234.37 | 3497.47 |
| Polygon-295 | 210.05 | 2609.64 |
| Polygon-296 | 374.79 | 9650.75 |
| Polygon-297 | 247.82 | 4263.75 |
| Polygon-298 | 314.84 | 6385.62 |
| Polygon-299 | 285.62 | 5040.79 |
| Polygon-300 | 240.46 | 3852.91 |
| Polygon-301 | 297.41 | 5980.94 |
| sum of areas of occluded dentinal tubules | | 528519.86 |
| Polygon-302 | 215.44 | 3000.47 |
| Polygon-303 | 252.60 | 4263.75 |
| Polygon-304 | 282.44 | 5505.48 |
| Polygon-305 | 216.98 | 2378.83 |
| Polygon-306 | 322.17 | 6657.97 |
| Polygon-307 | 306.08 | 6585.65 |
| Polygon-308 | 263.72 | 4448.39 |
| Polygon-309 | 279.87 | 5305.45 |
| Polygon-310 | 246.73 | 3768.28 |
| Polygon-311 | 286.02 | 5473.17 |
| Polygon-312 | 354.67 | 8310.53 |
| Polygon-313 | 363.00 | 8196.67 |
| Polygon-314 | 408.89 | 9643.05 |
| Polygon-315 | 248.77 | 4025.25 |
| Polygon-316 | 368.62 | 9359.93 |
| Polygon-317 | 323.47 | 7438.09 |
| Polygon-318 | 292.28 | 5403.92 |
| Polygon-319 | 271.70 | 3914.46 |
| Polygon-320 | 394.72 | 9681.52 |
| Polygon-321 | 354.14 | 8138.20 |
| Polygon-322 | 414.01 | 8116.66 |
| Polygon-323 | 382.69 | 8592.12 |
| Polygon-324 | 235.94 | 3680.58 |
| Polygon-325 | 498.65 | 14279.16 |

(continued)

| Results of image analysis of FIG. 3 | | |
|---|---|---|
| Measurement Table | | |
| Name | Length | Area |
| Polygon-326 | 361.47 | 8696.75 |
| Polygon-327 | 262.74 | 4683.81 |
| Polygon-328 | 451.88 | 11234.07 |
| Polygon-329 | 284.19 | 5257.75 |
| Polygon-330 | 452.27 | 12431.18 |
| Polygon-331 | 395.81 | 8901.40 |
| Polygon-332 | 278.91 | 4829.99 |
| Polygon-333 | 289.58 | 4700.74 |
| Polygon-334 | 371.75 | 9207.60 |
| Polygon-335 | 296.64 | 5442.39 |
| Polygon-336 | 271.23 | 5117.73 |
| Polygon-337 | 257.83 | 3492.86 |
| Polygon-338 | 346.80 | 7675.05 |
| Polygon-339 | 377.10 | 9086.04 |
| Polygon-340 | 219.24 | 3137.42 |
| Polygon-341 | 196.39 | 2509.62 |
| Polygon-342 | 309.22 | 6401.00 |
| Polygon-343 | 251.85 | 3125.11 |
| Polygon-344 | 340.13 | 8018.18 |
| Polygon-345 | 269.97 | 4893.08 |
| Polygon-346 | 368.74 | 9178.36 |
| Polygon-347 | 351.72 | 6630.27 |
| Polygon-348 | 300.95 | 5645.50 |
| Polygon-349 | 276.33 | 5240.82 |
| Polygon-350 | 416.53 | 11217.14 |
| Polygon-351 | 330.51 | 6788.76 |
| Polygon-352 | 372.76 | 8053.57 |
| Polygon-353 | 338.13 | 7662.74 |
| Polygon-354 | 257.23 | 4131.42 |
| Polygon-355 | 255.32 | 3989.86 |
| Polygon-356 | 315.56 | 6716.44 |
| Polygon-357 | 283.78 | 5533.18 |
| Polygon-358 | 287.60 | 5508.56 |
| Polygon-359 | 403.60 | 11095.59 |
| Polygon-360 | 228.20 | 3131.26 |
| Polygon-361 | 344.66 | 8181.28 |

(continued)

| Results of image analysis of FIG. 3 | | |
|---|---|---|
| Measurement Table | | |
| Name | Length | Area |
| Polygon-362 | 291.98 | 5331.61 |
| Polygon-363 | 295.80 | 5562.41 |
| Polygon-364 | 292.55 | 4940.78 |
| Polygon-365 | 295.29 | 5945.55 |
| Polygon-366 | 273.68 | 4993.09 |
| Polygon-367 | 321.75 | 6650.27 |
| Polygon-368 | 324.58 | 6861.08 |
| Polygon-369 | 322.02 | 6327.15 |
| Polygon-370 | 305.11 | 6024.02 |
| Polygon-371 | 350.56 | 7708.90 |
| Polygon-372 | 291.09 | 5691.66 |
| Polygon-373 | 290.71 | 5387.00 |
| Polygon-374 | 288.15 | 5533.18 |
| Polygon-375 | 394.22 | 8090.50 |
| Polygon-376 | 364.98 | 7293.45 |
| Polygon-377 | 280.06 | 4596.11 |
| Polygon-378 | 263.51 | 4514.55 |
| Polygon-379 | 282.67 | 5263.90 |
| Polygon-380 | 247.42 | 3512.86 |
| Polygon-381 | 308.31 | 5691.66 |
| Polygon-382 | 318.48 | 6691.82 |
| Polygon-383 | 274.44 | 5056.18 |
| Polygon-384 | 248.23 | 3795.98 |
| Polygon-385 | 327.14 | 7071.88 |
| Polygon-386 | 339.19 | 7770.45 |
| Polygon-387 | 252.82 | 4216.05 |
| sum of areas of total dentinal tubules | | 544236.19 |

[Table 2]

| Results of image analysis of FIG. 3 | | |
|---|---|---|
| Measurement Table | | |
| Name | Length | Area |
| Polygon-105 | 237.82 | 3508.24 |
| Polygon-106 | 221.03 | 2922.00 |
| Polygon-107 | 344.27 | 7325.76 |
| Polygon-108 | 213.22 | 2874.30 |

(continued)

| Results of image analysis of FIG. 3 | | |
| Measurement Table | | |
| Name | Length | Area |
| --- | --- | --- |
| Polygon-109 | 264.51 | 3843.68 |
| Polygon-110 | 299.96 | 6065.57 |
| Polygon-111 | 309.73 | 6616.42 |
| Polygon-112 | 182.01 | 1737.20 |
| Polygon-113 | 221.94 | 3362.07 |
| Polygon-114 | 248.20 | 4099.10 |
| Polygon-115 | 341.45 | 6124.04 |
| Polygon-116 | 238.74 | 3785.21 |
| Polygon-117 | 283.24 | 5316.22 |
| Polygon-118 | 204.15 | 2546.55 |
| Polygon-119 | 220.49 | 3314.37 |
| Polygon-120 | 232.73 | 2965.08 |
| Polygon-121 | 280.88 | 5260.83 |
| Polygon-122 | 334.37 | 7573.50 |
| Polygon-123 | 307.32 | 6459.48 |
| Polygon-124 | 283.19 | 5148.50 |
| Polygon-125 | 246.95 | 4092.95 |
| Polygon-126 | 252.26 | 4046.79 |
| Polygon-127 | 276.86 | 5328.53 |
| Polygon-128 | 312.66 | 6265.60 |
| Polygon-129 | 268.04 | 5051.56 |
| Polygon-130 | 258.33 | 3845.22 |
| Polygon-131 | 305.04 | 6039.41 |
| Polygon-132 | 320.61 | 6496.40 |
| Polygon-133 | 328.62 | 6914.93 |
| Polygon-134 | 252.69 | 4279.13 |
| Polygon-135 | 249.73 | 4272.98 |
| Polygon-136 | 394.33 | 8088.96 |
| Polygon-137 | 386.02 | 7073.42 |
| Polygon-138 | 320.74 | 6694.90 |
| Polygon-138 | 269.35 | 5010.02 |
| Polygon-140 | 261.90 | 4851.53 |
| Polygon-141 | 348.20 | 8264.37 |
| Polygon-142 | 268.01 | 5125.42 |
| Polygon-143 | 289.66 | 5013.09 |
| Polygon-144 | 299.87 | 5762.44 |

(continued)

| Results of image analysis of FIG. 3 | | |
|---|---|---|
| Measurement Table | | |
| Name | Length | Area |
| Polygon-145 | 306.32 | 5636.27 |
| Polygon-146 | 286.29 | 5448.55 |
| Polygon-147 | 227.03 | 3466.70 |
| Polygon-148 | 236.72 | 3894.46 |
| Polygon-149 | 277.87 | 5176.20 |
| Polygon-150 | 241.39 | 3948.31 |
| Polygon-151 | 268.62 | 4474.55 |
| Polygon-152 | 415.82 | 10612.43 |
| Polygon-153 | 328.76 | 6731.83 |
| Polygon-154 | 272.13 | 4282.21 |
| Polygon-155 | 322.70 | 6685.66 |
| Polygon-156 | 301.12 | 5840.92 |
| Polygon-157 | 336.68 | 6031.72 |
| Polygon-158 | 277.37 | 5188.51 |
| Polygon-159 | 302.85 | 5259.29 |
| Polygon-160 | 249.77 | 4305.29 |
| Polygon-161 | 297.37 | 6451.78 |
| Polygon-162 | 231.71 | 3771.36 |
| Polygon-163 | 293.60 | 5266.98 |
| Polygon-164 | 289.86 | 5550.10 |
| Polygon-165 | 267.13 | 4923.85 |
| Polygon-166 | 264.71 | 4039.10 |
| Polygon-167 | 190.88 | 1754.12 |
| Polygon-168 | 256.12 | 4331.45 |
| Polygon-169 | 264.67 | 4677.66 |
| Polygon-170 | 348.99 | 6787.22 |
| Polygon-171 | 357.38 | 8802.92 |
| sum of areas of total dentinal tubules | | 346705.21 |
| Polygon-172 | 274.37 | 4651.50 |
| Polygon-173 | 285.30 | 5528.56 |
| Polygon-174 | 227.13 | 3280.51 |
| Polygon-175 | 230.44 | 2737.35 |
| Polygon-176 | 331.01 | 6165.58 |
| Polygon-177 | 296.43 | 6127.12 |
| Polygon-178 | 357.22 | 7184.20 |
| Polygon-179 | 263.70 | 4633.03 |

(continued)

| Results of image analysis of FIG. 3 | | |
| --- | --- | --- |
| Measurement Table | | |
| Name | Length | Area |
| Polygon-180 | 239.94 | 3891.38 |
| Polygon-181 | 335.66 | 5143.88 |
| Polygon-182 | 301.30 | 5762.44 |
| Polygon-183 | 365.98 | 8336.69 |
| Polygon-184 | 305.49 | 5576.26 |
| Polygon-185 | 280.58 | 4866.92 |
| Polygon-186 | 277.35 | 5205.43 |
| Polygon-187 | 308.89 | 6264.06 |
| Polygon-188 | 259.76 | 4757.67 |
| Polygon-189 | 283.15 | 5408.54 |
| Polygon-190 | 331.91 | 6641.04 |
| Polygon-191 | 247.80 | 3234.35 |
| Polygon-192 | 252.29 | 3860.61 |
| Polygon-193 | 325.76 | 6151.73 |
| Polygon-194 | 236.60 | 3719.05 |
| Polygon-195 | 373.20 | 6687.20 |
| Polygon-196 | 307.30 | 4808.45 |
| Polygon-197 | 328.88 | 6936.47 |
| Polygon-198 | 319.35 | 5603.96 |
| Polygon-199 | 283.92 | 5286.98 |
| sum of areas of occluded dentinal tubules | 148450.96 | |

**INDUSTRIAL APPLICABILITY**

As described above, the composition for prevention of tooth hypersensitivity according to the invention can rapidly show the effect of preventing tooth hypersensitivity while maintaining the sensory feel of conventional tooth desensitizers, and it can maintain the tooth hypersensitivity-preventing effect for a long time.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

**Claims**

1. A composition for prevention of tooth hypersensitivity comprising an adhesive polymer for coating teeth, an active ingredient for prevention of hypersensitivity, a substance for promoting the penetration of the active ingredient for prevention of hypersensitivity, a desensitizing active ingredient, and a pH-adjusting agent.

2. The composition according to claim 1, wherein the composition for prevention of tooth hypersensitivity preferably comprises, based on 100 parts by weight of the adhesive polymer for coating teeth, 2-3 parts by weight of the active ingredient for prevention of hypersensitivity, 2-10 parts by weight of the substance for promoting the penetration of the active ingredient for prevention of hypersensitivity, 4-10 parts by weight of the desensitizing ingredient and 2-3 parts by weight of the pH-adjusting agent.

3. The composition according to claim 1, the adhesive polymer for coating teeth is one or more selected from the group consisting of hydroxypropyl methyl cellulose, hydroxyethyl cellulose, polyvinyl alcohol, polyvinyl pyrrolidone, hydroxypropyl cellulose, polyacrylate derivative carbomer, a copolymer of methyl vinyl ether and maleic anhydride, and polyethylene oxide.

4. The composition according to claim 3, wherein the polyvinyl pyrrolidone is one or more selected from the group consisting of polyvinyl pyrrolidone K-12, polyvinyl pyrrolidone K-15, polyvinyl pyrrolidone K-17, polyvinyl pyrrolidone K-25, polyvinyl pyrrolidone K-30, polyvinyl pyrrolidone K-60, polyvinyl pyrrolidone K-90 and polyvinyl pyrrolidone K-120.

5. The composition according to claim 1, wherein the active substance for prevention of hypersensitivity is one or more selected from the group consisting of potassium nitrate, stannous fluoride ($SnF_2$), sodium monofluoride phosphate (MFP), sodium fluoride (NaF), Sodium silicofluoride ($Na_2SiF_6$), calcium hydroxide ($Ca(OH)_2$), potassium oxalate ($K_2C_2O_4$) and strontium chloride ($SrCl_2$).

6. The composition according to claim 1, wherein the substance for promoting the penetration of the active ingredient for prevention of hypersensitivity is a peptide having an arginine of 80-90%.

7. The composition according to claim 6, wherein the peptide having an arginine content of 80-90% is one or more selected from the group consisting of TAT (trans-activator of transcription), penetratine, polylysine, polyarginine and protamine fragments.

8. The composition according to claim 1, wherein the desensitizing active ingredient is one or more selected from the group consisting of salts, potassium nitrate and strontium chloride.

9. The composition according to claim 8, wherein the salts are selected from the group consisting of potassium hydroxide, magnesium hydroxide, sodium chloride, calcium phosphate, silver nitrate and sodium citrate.

10. The composition according to claim 1, wherein the pH-adjusting agent is one or more selected from the group consisting of disodium phosphate, trisodium phosphate, sodium pyrophosphate, potassium pyrophosphate, sodium hydrogen carbonate, potassium hydroxide, ammonium hydroxide, triethanolamine, citric acid, sodium citrate, and sodium hydroxide.

11. The composition according to claim 1, wherein the composition for prevention of tooth hypersensitivity has a pH of 4.0-11.0.

12. The composition according to claim 1, wherein the composition for prevention of tooth hypersensitivity further comprise a sweetening agent and/or a flavoring agent.

13. The composition according to claim 12, wherein the sweetening agent is preferably one or more selected from the group consisting of sorbitol, mannitol, xylitol, stevioside and saccharine.

14. The composition according to claim 12, wherein the flavoring agent is one or more selected from the group consisting of menthol, peppermint oil, L-menthol and cinnamic acid.

15. The composition according to claim 1, wherein the composition for prevention of tooth hypersensitivity is in the form of gel or liquid.

16. A method for preparing a composition for prevention of tooth hypersensitivity of claim 1, comprising the steps of: comprising the steps of:

(a) dissolving in a volatile solvent an adhesive polymer for coating teeth to form an adhesive polymer solution;
(b) mixing the adhesive polymer solution of step (a) with an active ingredient for prevention of hypersensitivity, a substance for promoting the penetration of the active ingredient for prevention of hypersensitivity and a desensitizing active ingredient to form a mixture;
(c) adding a pH-adjusting agent to the mixture of step (b) to adjust the pH of the mixture; and

(d) filtering the pH-adjusted mixture of step (c) to remove impurities.

17. The method according to claim 16, wherein the volatile solvent that is used in step (a) of the inventive method is one or more selected from the group consisting of ethanol, methanol and butanol.

FIG. 1

FIG. 2

FIG. 3

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 2134862
- US 3122483 A
- US 4357318 A

### Non-patent literature cited in the description

- Journal of Periodontology. **John W. Federn.** American East Area. 1997, vol. 25
- **Bareire Green et al.** *American Journal of Periodontology,* October 1977, vol. 48 (10
- **Willard J. Tablet et al.** *American Journal of Periodontology,* September 1980, vol. 51 (9
- **Milton Hodosh.** *Journal of the American Dental Association,* April 1974, vol. 88